Europäisches Patentamt

European Patent Office

Office européen des brevets

(11) Veröffentlichungsnummer: **0 275 109**
**A2**

(12) **EUROPÄISCHE PATENTANMELDUNG**

(21) Anmeldenummer: 88100476.6

(22) Anmeldetag: 14.01.88

(51) Int. Cl.⁴: **A 61 K 7/48**

(30) Priorität: 15.01.87 DE 3701036

(43) Veröffentlichungstag der Anmeldung:
20.07.88 Patentblatt 88/29

(84) Benannte Vertragsstaaten: **BE DE FR GB**

(71) Anmelder: **Heyl Chemisch-pharmazeutische Fabrik
GmbH & Co. KG
Goerzallee 253
D-1000 Berlin 37 (DE)**

(72) Erfinder: **Bunte, Thomas, Dr.
Gustav-Müller-Strasse 31
D-1000 Berlin 62 (DE)**

**Parr, Wolfgang, Dr. Dipl.-Chem.
Eitel-Fritz-Strasse 16
D-1000 Berlin 37 (DE)**

**Heyl, Eduard, Dr.
Limastrasse 11
D-1000 Berlin 37 (DE)**

**Heinzel, Wolfgang, Dr. Dipl.-Chem.
Eppinger Strasse 19
D-1000 Berlin 33 (DE)**

(74) Vertreter: **Kinzebach, Werner, Dr. et al
Patentanwälte Reitstötter, Kinzebach & Partner
Sternwartstrasse 4 Postfach 86 06 49
D-8000 München 86 (DE)**

(54) **Kosmetisches Mittel.**

(57) Die Erfindung betrifft kosmetische Mittel, die als Wirkstoff ein makromolekulares Glykoprotein (Undulin), dessen Fragmentierungsprodukte oder ein Gemisch von Undulin, Prokollagen und Kollagen Typ I enthalten.

Die erfindungsgemäßen Mittel wirken dem Altern der Haut entgegen und halten sie jung und elastisch.

EP 0 275 109 A2

**0 275 109**

**Beschreibung**

<u>Kosmetisches Mittel</u>

Die Erfindung betrifft kosmetische Mittel, die das makromolekulare Glykoprotein "Undulin", dessen Fragmentierungsprodukte oder ein Gemisch von Prokollagen, Kollagen Typ I und Undulin enthalten.

Interstitielles Bindegewebe besteht zur Hauptsache aus Kollagen-I-Fibrillen. Ultrastrukturelle Studien lassen vermuten, daß Makromoleküle wie z.B. Fibronektin und Proteoglykan den Zusammenhalt der Kollagenfibrillen vermitteln. Bei der Suche nach weiteren Strukturkomponenten für die supramolekulare Organisation der Kollagen-I-Fibrillen wurde bei der Extraktion von Häuten fötaler oder neugeborener Säugetiere das Protein Undulin erhalten, das eine wesentliche Rolle beim Zusammenhalt der Kollagen-I-Fibrillen spielt und das wertvolle kosmetische Eigenschaften hat.

Der Alterungsprozeß des menschlichen Organismus zeigt sich am deutlichsten an der Haut, insbesondere an der lichtbestrahlten Haut, z.B. im Gesicht und an den Händen. Diese physiologische Alterung der Haut findet hauptsächlich im Korium, der Lederhaut, statt. Mit zunehmendem Alter versteifen die kollagenen Fasern der Haut, z.B. durch intermolekulare Vernetzungen, wobei das Wasserbindungsvermögen vermindert wird. Diese sich im kutanen Bindegewebe abspielenden Altersveränderungen führen zu einem allmählichen Elastizitäts- und Turgorverlust der Haut, der wiederum eine verstärkte Falten- und Runzelbildung der Haut zur Folge hat.

Diesen Prozeß zu verlangsamen, eine echte Revitalisierung der Haut zu erreichen, ist oberstes Ziel aller kosmetischen Bemühungen. Hierzu sind bereits kollagenhaltige Prä parate vorgeschlagen worden, man vgl. z.B. die DE-OSen 25 14 844, 25 17 452, 24 62 221, 20 64 604 oder 30 46 133. Die Wirkung der bekannten Präparate ist jedoch nicht zufriedenstellend.

Der Erfindung liegt die Aufgabe zugrunde kosmetische Mittel bereitzustellen, welche mit Hilfe eines bestimmten makromolekularen Glykoproteins den Zustand der Haut und des Keratins verbessern und revitalisierend wirken.

Gelöst wird diese Aufgabe durch Mittel, die in einem üblichen kosmetischen Träger oder Verdünnungsmittel einzeln oder in Mischung das Glykoprotein "Undulin", dessen Fragmentierungsprodukte oder ein Gemisch von Undulin, Prokollagen und Kollagen Typ I enthalten.

Undulin ist ein stark aggregiertes, makromolekulares Glykoprotein, das hauptsächlich mit einem Molekulargewicht von ≥ 1 000 000 vorliegt, ermittelt anhand von SDS-Polyacrylamid-Gelelektrophorese unter nicht-reduzierenden Bedingungen. Unter Verwendung eines Kollagen-I-Standards sind nach Reduktion eine charakteristische Bande bei 270 000 und ein Dublett bei 175 000/180 000 anfärbbar. Die Mobilität der Dublettbande ist deutlich größer als die des reduzierten Fibronektins.

Undulin ist nach Lyophilisation praktisch unlöslich in nicht-denaturierenden Puffersystemen, z.B. Tris•HCl Phosphat-Puffer oder verdünnter Essigsäure. Dagegen ist Undulin in 6 M Guanidin-HCl, pH 7 bis 8, in Gegenwart von Proteaseinhibitoren gut löslich. In Assoziation mit Kollagen-I läßt sich Undulin in 0,1 M Essigsäure/1 % Triton-X-100 in Lösung bringen.

Derartige Präparationen lassen sich nach Kegelbedampfung elektronenmikroskopisch untersuchen. Dabei erscheint das Molekül in Form einer bisher unbekannten Struktur, die aus einem globulären Kopfanteil mit einem Durchmesser von ca. 12 nm und einem ca. 100 nm langen Schwanzteil besteht. Häufig werden aggregierte Formen, die sich aus zwei, vier oder mehreren dieser Strukturen zusammensetzen und die oft mit Kollagen-I-Molekülen assoziiert sind, beobachtet.

In der Aminosäureanalyse (6 M HCl, 24 Stunden, unter Stickstoff bei 110°C) ergibt Undulin eine für große Glykoproteine typische Aminosäurezusammensetzung mit einem hohen Anteil an hydrophoben und sauren Aminosäuren.

Die Aminosäurezusammensetzung ist aus der nachfolgenden Tabelle ersichtlich.

2

## Tabelle 1: Aminosäureanalyse von Undulin

| 4-Hyp | – | Met | 5 |
|-------|-----|-----|-----|
| Asx | 98 | Ile | 47 |
| Thr | 67 | Leu | 80 |
| Ser | 83 | Tyr | 27 |
| Glx | 113 | Phe | 37 |
| Pro | 57 | His | 28 |
| Gly | 79 | Hyl | – |
| Ala | 75 | Lys | 58 |
| Cys | 25 | Arg | 43 |
| Val | 74 | | |

Undulin ist in der EP-A-86 904 796 beschrieben, auf deren Offenbarungsgehalt hiermit in vollem Umfang Bezug genommen wird.

Undulin und dessen Fragmentierungsprodukte sind dadurch erhältlich, daß man in Gegenwart von Proteaseinhibitoren

    A) Häute von fötalen oder neugeborenen Säugetieren oder Rinderplacenta einer Neutralsalzextraktion unterzieht,

    B) den Extrakt einer fraktionierten Salzfällung unterzieht,

    C) das Präzipitat löst, dialysiert und gegebenenfalls durch DEAE-Chromatographie reinigt, wobei man Undulin als Rohprodukt erhält.

    D) das Rohprodukt extrahiert, gewünschtenfalls dialysiert und lyophilisiert,

    E) das Undulin zur Herstellung der Fragmentierungsprodukte mit neutralen oder sauren Proteasen spaltet, bis man Fragmente mit einem Molekulargewicht < 400 000 erhält, und

    F) das gemäß Stufe E erhaltene Produkt mittels CM- und DEAE-Cellulose-Chromatographie, Molekularsieb und/oder HPLC in einem Puffer von pH 3,6 - 8,2 reinigt.

Vorzugsweise erfolgt die Extraktion gemäß Stufe A), indem man zunächst eine Vorextraktion mit Neutralpuffer zur Entfernung von Serumproteinen und Lipiden durchführt. Dazu verwendet man einen 10 - 200 mM Neutralpuffer, beispielsweise 50 - 200 mM Tris • HCl, 4 - 5 M NaCl vom pH 7 - 8, 20 - 200 mM HEPES- oder PIPES-Puffer{2-[4-(2-Hydroxyethyl)-1-piperazinyl]-ethansulfonsäure bzw. Piperazin-N,N'-bis(2-ethansulfonsäure}, zusammen mit 0,1 - 2 % eines nicht-ionischen Detergens, beispielsweise Triton X-100. Man verwendet etwa 2 - 4 l Puffer pro kg Haut (Feuchtgewicht). Daran schließt sich die eigentliche Extraktion an, die vorzugsweise im gleichen Puffersystem und mit der gleichen Menge erfolgt, jedoch bei niedrigerer Ionenstärke (0,3 bis 0,8 M NaCl, insbesondere 0,5 M NaCl), durchgeführt wird.

Der Extrakt wird anschließend gemäß Stufe B) einer fraktionierten Salzfällung unterzogen. Im ersten Schritt wird der Extrakt durch Zugabe von festem Ammoniumsulfat gefällt, bis ein Sättigungsgrad von 20 bis 50 % erreicht ist. Das so erhaltene Präzipitat wird dann erneut in 10 - 200 mM Neutralpuffer des oben angegebenen Typs (0,3 - 0,8 M NaCl, insbesondere 0,5 M NaCl) gelöst, und einem fraktionierten Salzfällung mit einem Alkalihalogenid, beispielsweise NaCl, KCl, unterworfen. Die Hauptmenge des Undulins wird zwischen 1,5 M und 4,5 M gefällt.

Vorzugsweise erfolgt die Salzfällung durch aufeinanderfolgende Zugabe von 1.6, 2.6 und 4.5 M Salzlösungen.

Bei Verwendung von Rinderplacenta als Ausgangsmaterial kann die fraktionierte Salzfällung entfallen.

Zur Reinigung wird das gemäß Stufe B) erhaltene Präzipitat in Neutralpuffer/1-4 M Harnstoff/0,25-0,5 M NaCl gelöst und mit DEAE-Cellulose behandelt. Die ungebundene Fraktion wird gegen den gleichen Puffer, der jedoch nur 0,01 - 0,05 M NaCl enthält, dialysiert. Das so erhaltene Präzipitat be steht zu ca. 70 % aus Undulin. Der Überstand enthält weiteres Undulin, das man durch Chromatographie an DEAE-Cellulose, wobei man einen Salzgradienten (z.B. 0,2 - 0,3 M NaCl als Elutionsmittel verwendet, in mehr als 90%iger Reinheit gewinnen kann.

Das 70%ige Präparat kann durch Extraktion mit Neutralpuffer oder 0,05 bis 0,5 M Essigsäure, gegebenenfalls in Gegenwart eines nicht-ionischen Detergens, von Verunreinigungen befreit werden, wobei man ein Produkt mit einer Reinheit von mehr als 90 % erhält.

Als weitere Reinigungsoperation kann sich eine Dialyse, beispielsweise gegen Essigsäure (0,01 bis 1 M, insbesondere 0,1 M) anschließen. Die Präparate werden dabei insbesondere von Salzen befreit. Anschließend werden die Präparate lyophilisiert.

Durch gegebenenfalls wiederholte Anwendung dieser Schritte erhält man homogenes und reines Undulin. Die Ausbeute beträgt ca. 200 bis 300 mg/kg Haut (Feuchtgewicht).

Weiter läßt sich Undulin noch reinigen, wie unten für die Fragmentierungsprodukte des Undulins beschrieben.

Das gesamte Verfahren wird zweckmäßigerweise bei Kühlraumtemperaturen (4 - 10°C) und in Gegenwart von Proteaseinhibitoren, beispielsweise Phenylmethylsulfonylfluorid (PMSF), N-Ethylmaleimid (NEM), Ethylendiamintetraessigsäure (EDTA), Sojabohnen-Trypsininhibitor (SBTI), Trasylol und Benzamidinhydrochlorid, durchgeführt.

Undulin ist sehr sensitiv gegenüber neutralen oder sauren Proteasen und läßt sich mit diesen in Fragmentierungsproduke überführen. Geeignete neutrale Proteasen, deren pH-Optimum im allgemeinen im Bereich von 6 bis 8 liegt, sind z.B. Trypsin, Thrombin, Plasmin, Elastase, Kathepsin, V8-Staphylokokkenprotease, Chymotrypsin, Papain und Thermolysin. Beispiele geeigneter saurer Proteasen, deren pH-Optimum im Bereich von 3 bis 5 liegt, sind z.B. Pepsin, saures Kathepsin, Chymosin etc.

Bevorzugte Proteasen sind Trypsin, Elastase, Plasmin, Thrombin, V8-Staphylokokkenprotease und Chymotrypsin, sowie Pepsin.

Die Proteolyse erfolgt in geeigneten Medien, deren pH-Wert dem pH-Optimum der Proteasen entspricht. Geeignete Medien für neutrale Proteasen sind Neutralpuffer. z.B. Ammoniumbicarbonat/Essigsäure. Ein geeignetes Medium für saure Proteasen ist beispielsweise 0,1 bis 1 M Essigsäure.

Das Enzym : Substratverhältnis liegt in der Regel im Bereich von 1 : 3 bis 1 : 100, vorzugsweise 1 : 10 bis 1 : 50.

Proteasefragmente von Undulin mit einem Molekulargewicht von < 400 000, vorzugsweise < 100 000 sind in neutralen Puffern (z.B. 50 mM Tris, 100 mM NaCl pH 6 - 8) löslich. Auch diese Fragmente sind kosmetisch brauchbar.

So liefert beispielsweise der Abbau des Undulins mit Trypsin bei 30 °C über Zwischenstufen mit einem Molekulargewicht von 180 000, 120 000 und 90 000 ein Hauptprodukt mit einem Molekulargewicht von 60 000. Das Endprodukt des Pepsinabbaus bei 4 °C besitzt ein Molekulargewicht von 45 000.

Eine proteolytische Fragmentierung von Undulin erfolgt auch, wenn man lyophilisiertes Undulin in Neutralpuffer ohne Proteaseinhibitoren gibt (Autoproteolyse). Führt man beispielsweise eine 20 bis 30-stündige (insbesondere 24-stündige) Inkubation von Undulin bei 0 bis 5 °C in Neutralpuffer des oben angegebenen Typs bzw. in Ammoniumbicarbonat/Essigsäure-Puffer vom pH 7,0 ohne Proteaseinhibitoren durch, so erhält man im wesentlichen zwei Fragmente mit einem Molekulargewicht von 50 000 und 70 000 D.

Wenn man diese Inkubation dagegen bei 33 bis 38°C (insbesondere 37 °C) durchführt, so erhält man Fragmente mit Molekulargewichten zwischen 10 000 und 70 000 D.

Die Fragmente lassen sich mittels CM- und DEAE-Cellulose-Chromatographie, Molekularsieb und/oder HPLC in üblichen Puffern mit einem pH von 3,6 bis 8,2 reinigen. Geeignete saure Puffer sind beispielsweise Acetat- und Zitratpuffer. Geeignete Neutralpuffer wurden bereits oben erwähnt und geeignete alkalische Puffer sind Ammoniumbicarbonat, Natriumacetat.

Es ist bekannt, daß Kollagenmoleküle zunächst als sogenannte Vorläufermoleküle (Prokollagen) im Organismus gebildet werden. Diese Vorläufermoleküle verfügen sowohl am C- als auch am N-Terminus über zusätzliche Aminosäuren. Diese zusätzlichen Aminosäuren werden bei einem Teil der Prokollagenmoleküle extracellulär durch spezifische Proteasen beseitigt. Anschließend findet dann in der extracellulären Matrix die Kollagenfibrillenformation durch Selfassembly statt. Die so geformten Fibrillen werden anschließend stabilisiert durch kovalente Crosslinks in und zwischen Kollagenmolekülen.

Es wurde nun gefunden, daß Undulin für die Erhaltung der supramolekularen Struktur der oben genannten Fibrillen in hohem Maße wichtig ist. Diese Kollagen-Undulinfibrillen sind verantwortlich für die Elastistizität und Oberflächenstruktur der Haut.

Es ist bekannt, daß in der Haut älterer Menschen eine veränderte Kollagenfibrillenformation stattfindet, die zu einem Elastizitätsverlust der Haut führt. Aus wissenschaftlichen Untersuchungen kann der Schluß gezogen werden, daß dieses an einem Mangel von Prokollagenmolekülen liegt. Prokollagen scheint eine wichtige Komponente dafür zu sein, daß die Elastizität und Oberflächenstruktur der Haut erhalten bleibt. Durch Einarbeitung von Undulin, dessen Fragmentierungsprodukten oder des obigen undulinhaltigen Gemisches in kosmetische Mittel kann somit dem Altern der Haut entgegengewirkt und die Haut jung und elastisch gehalten werden.

Die erfindungsgemäßen kosmetischen Mittel können daher auch einen Extrakt enthalten, der im wesentlichen aus Undulin, Prokollagen und Kollagen Typ I besteht. Dieser Extrakt wird ebenfalls aus Häuten von fötalen oder neugeborenen Säugetieren oder aus Rinderplacenta gewonnen. Hierzu führt man zu nächst eine Neutralsalzextraktion wie oben für Undulin beschrieben, durch (Stufe A). Daran schließt sich eine fraktionierte Salzfällung an, bei der, wie für Undulin beschrieben, ein Ammoniumsulfatpräzipitat erzeugt und wieder in Neutralpuffer gelöst wird. Es folgt eine Salzfällung mit 1,5 bis 2,0 M, vorzugsweise 1,7 M, einer Alkalihalogenidlösung (NaCl, KCl). Anschließend wird die Salzkonzentration auf 4 bis 5 M Salz, insbesondere 4,5 M, erhöht. Das erhaltene Präzipitat wird anschließend erneut in Tris-Pl-0,1 bis 1,0 M Salz (vorzugsweise 0,5 M NaCl) gelöst und anschließend einer erneuten fraktionierten Salzfällung mit 1,5 bis 4,5 M unterworfen. Vorzugsweise erfolgt die Salzfällung durch Zugabe von 1.6, 2.6 und 4.5 M NaCl.

Das Präzipitat wird anschließend sorgfältig mit dem gleichen Puffer + 0,5 M NaCl (4.5 M NaCl, 50 mM Tris,

4

pH 7,4, 0.5 M NaCl) gewaschen. Das Präzipitat wird anschließend gegen 0,01 bis 0,5 M (vorzugsweise 0,1 M) Essigsäure dialysiert und lyophilisiert. Gewünschtenfalls kann das Produkt zur Einarbeitung in die kosmetischen Mittel in Natriumcitratpuffer (0,1 bis 0,5 M; pH 3,5 bis 3,7) gelöst werden.

Das wie oben beschrieben erhaltene Präparat ist für kosmetische Zwecke sehr geeignet. Es besteht im wesentlichen aus Undulin, Prokollagen und Kollagen 1.

Herstellung von Undulin

Die Haut von fötalen oder neugeborenen Affen, Kälbern oder Ratten wird eingeweicht, homogenisiert und in 100 mM Tris 4,5 M NaCl vom pH 7,4, das 2 mM PMSF, 4 mM NEM und 10 mM EDTA als Proteaseinhibitoren enthält, tiefgefroren. Alle Arbeiten erfolgen bei 4°C und in Gegenwart von Proteaseinhibitoren (PI), wenn Puffer verwendet werden, deren pH oberhalb von 4,0 liegt. Das Homogenisat wird zunächst mit 50 mM Tris/PI/4,5 M NaCl, pH 7,4 und 1 % Triton X-100 (31/kg Feuchtgewicht) extrahiert. Der Rückstand wird anschließend mit 50 mM Tris/PI/0.5 M NaCl, pH 7,4(31/kg Feuchtgewicht) extrahiert. Aus der überstehenden Flüssigkeit erhält man durch Zugabe von Ammoniumsulfat bis zu einem Sättigungsgrad von 30% (168 g/l) ein Präzipitat. Dieses wird erneut in Tris/PI/0,5 M NaCl (31/kg Feuchtgewicht) gelöst. Die Lösung unterwirft man einer fraktionierten Salzfällung mit NaCl bis zu Endkonzentrationen von 1,7, 2,6 und 4,5 M.

Die Fraktion bei 2,6 M NaCl wird in 50 mM Tris, PI, 0,3 M NaCl, 1 - 4 M Harnstoff, pH 7,4 gelöst und gegen dieses Gemisch dialysiert. Einen Niederschlag (Kollagen-I-Aggregat) zentrifugiert man ab und die überstehende Flüssigkeit rührt man mit DEAE-Cellulose. Die ungebundene Fraktion dialysiert man gegen Tris,PI, 0,02 M NaCl, pH 7,4, wobei man ein Präzipitat erhält, das aus Undulin, Kollagen I und einem Protein mit einem Molekulargewicht von 35 000 besteht. Das lösliche Material,chromatographiert man an DEAE-Cellulose, wobei man nach Elution mit einem Salzgradienten von ungefähr 0,25 M NaCl Undulin mit einer Reinheit von > 90 % erhält.

Die Undulin enthaltenen Fraktionen werden gegen 0,1 M Essigsäure dialysiert und lyophilisiert. Zur weiteren Reinigung wird das Lyophilisat mit 0,5 M Essigsäure, 1 % Triton X-100 bei 4°C extrahiert,wobei u.a. verunreinigende Proteine entfernt werden. Auf diese Weise hergestelltes Undulin ist homogen und rein, die Ausbeute beträgt 200 mg/kg eingesetzter Haut (Feuchtgewicht).

Trypsinabbau von Undulin

Das lyophilisierte Protein wird zu 1 mg/ml in 0,2 M $NH_4HCO_3$/Essigsäure, pH 7,0, suspendiert und mit Trypsin-TPCK (Serva, Heidelberg) im Enzym:Substrat-Verhältnis 1:50 bei 25°C unter Schütteln abgebaut. Aliquote werden nach 10,30,60 Minuten, 4 und 16 Stunden entnommen, mit Trypsininhibitor (Soybean Trypsin-Inhibitor, Serva, Heidelberg) versetzt und lyophilisiert. Der Abbau nach 16 Stunden enthält hauptsächlich ein Peptid vom Molekulargewicht 60 000. Die Reinigung erfolgt mittels DEAE-Cellulose-Chromatographie im Neutralpuffer (wie oben erwähnt). Anschließend wird gegen 0,1 M Essigsäure dialysiert unu lyophilisiert.

Pepsinabbau von Undulin

Für den Pepsinabbau wird Undulin zu 1 mg/1 ml in 0,5 M Essigsäure suspendiert und bei 4 bis 8 °C im Verhältnis 1 : 50 (Enzym zu Substrat) unter Schütteln mit Pepsin (Sigma, Taufkirchen) abgebaut. Nach 6 bis 10 Stunden erhält man als Hauptprodukt ein Fragment mit dem Molekulargewicht 45 000. Die Aufarbeitung und Reinigung erfolgt wie beim Trypsinabbau beschrieben.

Herstellung eines Extraktes, der im wesentlichen aus Undulin, Prokollagen und Kollagen Typ I besteht

Man arbeitet zunächst wie zuvor bei der Herstellung von Undulin beschrieben bis zum Lösen des Ammoniumsulfatpräzipitats. Das gelöste Ammoniumsulfatpräzipitat wird dann einer 1,7 M Salzfällung unterworfen. Anschließend wird die Salzkonzentration gleich auf 4,5 M NaCl erhöht. Das so erhaltene Präzipitat wird anschließend erneut gelöst in Tris-PI-0,5 M NaCl und nochmals fraktioniert mit 1.6, 2.6, 4.5 M NaCl. Nach extensiver Wäsche des Präzipitats mit 4,5 M NaCl, 50 mM Tris, pH 7,4, 0,5 M NaCl wird gegen 0.1 M Essigsäure dialysiert und lyophilisiert. Das Lyophilisat wird gegebenenfalls in Natriumcitratpuffer 0,13 M, pH 3,6 erneut gelöst.

Autoproteolyse von Undulin

Undulin wird zu 1 mg/ml in Neutralpuffer (0.2 M $NH_2CO_3$/Essigsäure, pH 7,0) ohne Proteaseinhibitoren gegeben und 24 Stunden bei 0 °C inkubiert. Man erhält Fragmente mit einem Molekulargewicht von 50 000 und 70 000 D. Die Aufarbeitung und Reinigung erfolgt wie beim Trypsinabbau beschrieben.

Die erfindungsgemäßen Mittel enthalten in der Regel etwa 0,2 bis 10 %, vorzugsweise etwa 0,5 bis 3 % Undulin, Undulinfragmente oder das oben beschriebene undulinhaltige Gemisch oder Mischungen davon. Die %-Angabe steht für Gewichtsprozent. bezogen auf das Gesamtgewicht des fertigen Mittels.

Der Wirkstoff wird in übliche kosmetische Grundlagen und Hilfsstoffe für z.B. Cremes, Gele, Milch oder Lotionen eingearbeitet.

Geeignete Grundlagen sind beispielsweise Öle, wie Vaselineöl, Avocadoöl, Paraffinöl und dergleichen. Brauchbare Hilfsstoffe sind beispielsweise Emulgatoren, wie Mischungen von Ölen und/oder Fettalkoholen oder polyäthoxylierten Alkoholen, Seifen und dergleichen; Verdickungsmittel, wie Natriumalgelat, Gummiarabikum, Xanthangummi, Cellulosederivate etc; Treibmittel zur Formulierung von Aerosolen, wie Kohlendioxid, Stickstoff; Lösungsmittel, wie Alkohole und dergleichen.

Die erfindungsgemäßen Mittel können auch üblicherweise in der Kosmetik eingesetzte Bestandteile enthalten. Dazu zählen beispielsweise Parfums, Farbstoffe, Konservierungsmittel, Antioxydantien, Sequestriermittel, weichmachende Mittel, Emulgatoren und dergleichen.

Die erfindungsgemäßen Mittel können auch kosmetisch aktive Additive enthalten. Dazu zählen z.B. Feuchthaltemittel, Karotinoide, Stabilisierungsmittel, Feuchtigkeitsregulatoren, pH-Regulatoren, UV-A- und UV-B-Filter etc.

Die nachfolgenden Beispiele erläutern die Erfindung. Als Wirkstoff können jeweils Undulin, ein Fragmentierungsprodukt oder das erwähnte undulinhaltige Gemisch oder Mischungen davon zur Anwendung kommen.

B e i s p i e l   1

Hautcreme:

| | | |
|---|---|---|
| Wirkstoff | 0,5 | g |
| Polyäthylencetyläther | 1,5 | g |
| Cetylalkohol | 2 | g |
| Vaselineöl | 6 | g |
| Avocadoöl | 4 | g |
| Lanolin | 4 | g |
| Parfüm, soviel wie erforderlich | | |
| steriles Wasser ad | 100 | g |

B e i s p i e l  2

Körpermilch:

| | | |
|---|---|---|
| Wirkstoff | 1 | g |
| Paraffinöl | 5 | g |
| Vaselinöl | 7 | g |
| Konservierungsmittel | 0,15 | g |
| Triäthanolaminstearat | 5 | g |
| Stearinsäure | 3 | g |
| steriles Wasser, ad | 100 | g |

B e i s p i e l  3

Hautlotion:

| | | |
|---|---|---|
| Wirkstoff | 1,5 | g |
| Konservierungsmittel | 0,15 | g |
| Polyvinylpyrrolidon | 3 | g |
| Äthanol | 20 | g |
| steriles Wasser, ad | 100 | g |

## B e i s p i e l   4

·Hautgel:

| | | |
|---|---|---|
| Wirkstoff | 1,5 | g |
| Konservierungsmittel | 0,15 | g |
| Äthanol | 40 | g |
| Propylenglykol | 42 | g |
| Acrylsäurepolymerisat (Carbopol 940 der Firma Goodrich Chemical Co.) | 1 | g |
| steriles Wasser, ad | 100 | g |

**Patentansprüche**

1. Kosmetisches Mittel,
**dadurch gekennzeichnet,** daß es als Wirkstoff Undulin, dessen Fragmentierungsprodukte oder einen Extrakt der Haut von fötalen oder neugeborenen Säugetieren der im wesentlichen aus Undulin, Prokollagen und Kollagen Typ I besteht, oder Mischungen davon enthält.

2. Kosmetisches Mittel nach Anspruch 1,
dadurch gekennzeichnet, daß es 0,2 bis 10 Gew.-% Wirkstoff, bezogen auf das Gesamtgewicht des Mittels, enthält.

3. Kosmetisches Mittel nach Anspruch 2,
dadurch gekennzeichnet, daß es 0,5 bis 3 Gew.-% Wirkstoff, bezogen auf das Gesamtgewicht des Mittels enthält.

4. Kosmetisches Mittel nach Anspruch 1,
dadurch gekennzeichnet, daß es als Creme, Gel, Milch oder Lotion vorliegt.

5. Kosmetisches Mittel nach Anspruch 1,
dadurch gekennzeichnet, daß es zusätzlich kosmetisch aktive Additive enthält, die ausgewählt sind aus der Gruppe, bestehend aus Feuchthaltemitteln, Karotinoiden, Stabilisierungsmitteln, Feuchtigkeitsregulatoren, pH-Regulatoren, Mitteln zur Modifizierung des osmotischen Druckes und UV-A- und UV-B-Filtern.

6. Gemisch aus Undulin, Prokollagen und Kollagen Typ I, dadurch erhältlich, daß man in Gegenwart von Proteaseinhibitoren

A) Häute von fötalen oder neugeborenen Säugetieren oder Rinderplacenta einer Neutralsalzextraktion unterzieht,

B) aus dem Extrakt mit Ammoniumsulfat ein Präzipitat erzeugt, das Präzipitat in Neutralpuffer löst und mit 1.5 bis 2,0 M Alkalihalogenidlösung fraktioniert fällt,

C) das Präzipitat erneut in Neutralpuffer löst und erneut mit einer 1,5 bis 4,5 M Salzlösung fällt und

D) das Präzipitat zur Reinigung extrahiert, dialysiert und lyophilisiert.